# EUROPEAN PATENT SPECIFICATION

(11) **EP 1 747 778 B1**
(45) Date of publication and mention of the grant of the patent: **20.10.2010**
(21) Application number: 05737195.7
(22) Date of filing: 09.05.2005
(51) Int. Cl.: A61K 31/205, A61K 31/122, A61P 43/00

(54) **ANTI-FATIGUE COMPOSITION**
ZUSAMMENSETZUNG GEGEN MÜDIGKEIT
COMPOSITION ANTIFATIGUE

(30) Priority: 11.05.2004 JP 2004141417; 10.09.2004 JP 2004263225
(43) Date of publication of application: 31.01.2007
(73) Proprietor: Kaneka Corporation, Kita-ku Osaka-shi, Osaka 530-8288 (JP)
(72) Inventor: KISHIDA, Hideyuki, 6750039 (JP); KAWABE, Taizo, 6728044 (JP); HOSOE, Kazunori, 6760025 (JP); FUJII, Kenji, Kobe-shi,Hyogo 6511202 (JP)
(74) Representative: Gillet, Raphaëlle
(86) International application number: PCT/JP2005/008422
(87) International publication number: WO 2005/107737

(56) References cited:
- EP-A- 1 588 703
- WO-A-98/43617
- WO-A-03/061395
- WO-A1-01/52822
- JP-A- 7 330 584
- JP-A- 10 109 933
- JP-A- 2003 119 127

## Description

### Technical Field

The present invention relates to a composition comprising a reduced coenzyme Q represented by the following formula (1) : 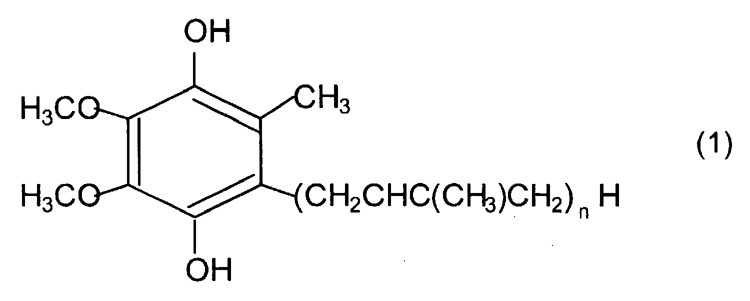 and carnitine, acetyl carnitine, acyl carnitine or proprionyl carnitine for use inpreventingor treating fatigue in a mammal, and where desired, an oxidized coenzyme Q represented by the following formula (2) : 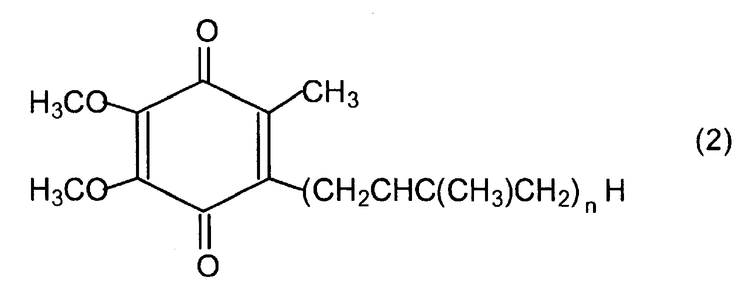 wherein n represents an integer of 1 to 12. Here, the anti-fatigue composition refers to a composition which can recover from or prevent physical fatigue due to exercise and physical fatigue during or after sickness or which can improve a tendency toward easily getting tired because of aging.

### Background Art

Heretofore, a lot of substances which exhibit an effect of alleviating fatigue have been reported. Carnitine derivatives are one of the examples and so is oxidized coenzyme Q.

Coenzyme Q is an essential component widely distributed in living bodies, from bacteria to mammals. In the case of human, coenzyme Q10 in which coenzyme Q has 10 repeating units in the side chain is known to be a main component. Coenzyme Q is a physiological component existing as a constituent of the electron transport system of mitochondria in cells of living bodies, and through repeated oxidation and reduction in living bodies, coenzyme Q functions as a transmitter in the electron transport system.

Coenzyme Q is known to have energy production activity, membrane stabilizing activity and antioxidation activity, and thus can be used for wide applications. Of the coenzymes Q, oxidized coenzyme Q (ubiquinone or ubidecarenone) is known to act effectively on the heart as is used for medical purposes as a congestive heart failure drug.

The effects reportedly include improvement of the oxygen utilization efficiency in cardiac muscle, activation of ATP production in cardiac muscle and improvement of cardiac function. In addition to such medical applications, the effects as nutrients and nutritional supplements have been reported like vitamins.

Japanese Patent Laid-Open No. 62-59208 discloses a composition for activating tissue metabolism which is composed of a mixture of ubiquinone and dry yeast powder, Japanese Patent Laid-Open No. 52-99220 discloses improvement of symptoms of myasthenia gravis, and Japanese Patent Laid-Open No. 52-99222 reports increase of erythrocytes. Further, effects of recovery from fatigue have been reported in Japanese Patent Laid-Open Nos. 7-330584, 7-330593 and 10-287560.

While carnitine derivatives have been generally used for improving gastrointestinal symptoms such as promotion of gastric secretion and gastric peristalsis, they have also attracted attention as an anti-fatigue substance because of the effect of promoting ATP synthesis by acting on mitochondria. Japanese Patent Laid-Open No. 7-330584 discloses that combination use of oxidized coenzyme Q₁₀ and a carnitine derivative leads to exertion of a synergistic anti-fatigue effect. However, there is no report that combination of reduced coenzyme Q and a carnitine derivative is synergistically effective for prevention of or recovery from fatigue.

WO 03/061395 discloses a ubiquinol-enriched fat containing food useful for fatigue induced by lack of ubiquinol.

WO 98/43617 discloses a dietary supplement comprising L-carnitine, oxidized coenzyme Q10 and taurine which is effective for cellular nutrition, mitochondrial energetics and function.

WO 01/52822 relates to storage stable compositions in dietary supplement, cosmetic or pharmaceutical dosage form comprising a reduced form of coenzyme Q in combination with a lipid solubme reducing agent effective to maintain coenzyme Q in its reduced state.

The present invention aims at providing an anti-fatigue composition safe and effective even at low doses.

### Disclosure of the Invention

The inventors of the present invention have conducted intensive studies and have found that by combining reduced coenzyme Q and carnitine, acetyl carnitine, acyl carnitine or proprionyl carnitine, a far greater synergistic anti-fatigue effect is exhibited compared to the effect brought about by the combination of oxidized coenzyme Q and a carnitine derivative. Japanese Patent Laid-Open No. 10-109933 discloses that co-existence of reduced coenzyme Q increases oral absorption of coenzyme Q compared to the case of using oxidized coenzyme Q alone. However, it is known that administration of oxidized coenzyme Q also leads to increase of reduced coenzyme Q due to reduction in living bodies, and it has been commonly understood that oxidized coenzyme Q and reduced coenzyme Q have similar effects on living bodies. Thus, the finding that a combination of carnitine, acetyl carnitine, acyl carnitine or proprionyl carnitine and reduced coenzyme Q is extremely effective for prevention of or recovery from fatigue far beyond the combination of oxidized coenzyme Q and carnitine, acetyl carnitine, acyl carnitine or proprionyl carnitine is extraordinary, and cannot be anticipated by those skilled in the art.

The inventors of the present invention have completed the present invention based on these findings. Accordingly, the present invention is as follows:
[1] An composition comprising
   (a) a reduced coenzyme Q represented by the following formula (1) : 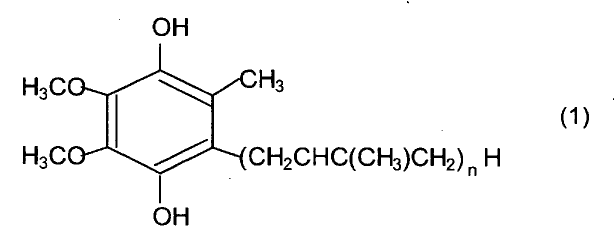 wherein n represents an integer of 1 to 12, and
   (b) carnitine, acetyl carnitine, acyl carnitine or proprionyl carnitine for use inpreventing or treating fatigue in a mammal;
[2] The composition according to [1], wherein the coenzyme Q is coenzyme Q₁₀;
[3] The composition according to [1] as a medicine or food;
[4] The composition according to [3], further comprising an antioxidant;
[5] The composition according to [4], wherein the antioxidant is selected from the group consisting of vitamin C, lycopene, vitamin A, carotenoids, vitamin B, flavonoids, polyphenols, glutathione, selenium, sodium thiosulfate, vitamin E, pycnogenol, flavangenol, pyrroloquinoline quinone, superoxide dismutase (SOD), glutathione peroxidase, glutathione-S-transferase, glutathione reductase, catalase and ascorbate peroxidase;
[6] Use of a composition comprising
   (a) a reduced coenzyme Q represented by the following formula (1) : 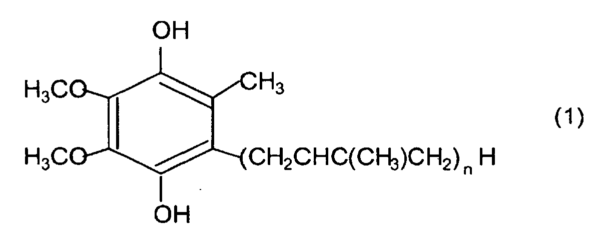 wherein n represents an integer of 1 to 12, and
   (b) carnitine , acetyl carnitine, acyl carnitine or proprionyl carnitine in the manufacture of a composition for preventing or treating fatigue in a mammal;

According to the present invention, by using reduced coenzyme Q and carnitine, acetyl carnitine, acyl carnitine or proprionyl carnitine in combination, an unexpectedly high anti-fatigue effect can be obtained. In particular, according to the present invention in which small doses of reduced coenzyme Q and carnitine, acetyl carnitine, acyl carnitine or proprionyl carnitine are used in combination, each of which has no effect when used alone, an anti-fatigue medicine or food which ensures reliable effects and safety can be provided.

### Best Mode for Carrying Out the Invention

Coenzyme Q is known to exist in living bodies, about 40 to 90% of which generally occurring in reduced form. The method of preparing reduced coenzyme Q is not particularly limited and for example, a method comprising producing coenzyme Q by a conventionally known method such as synthesis, fermentation or extraction from a natural source and concentrating the fraction of reduced coenzyme Q in eluted liquid using chromatography may be employed. In that case, it is also possible to add a common reducing agent such as sodium borohydride or sodium dithionite (sodium hydrosulfite) to the coenzyme Q if necessary, and after reducing the oxidized coenzyme Q contained in the above-mentioned coenzyme Q to form reduced coenzyme Q according to a usual method, subject the same to chromatography for concentration. Reduced coenzyme Q can also be obtained by a method in which the above-mentioned reducing agent is reacted with existing high purity coenzyme Q (oxidized coenzyme Q). Alternatively, microbial cells and the like which contain reduced coenzyme Q may also be used.

The proportion of the reduced form in coenzyme Q may be generally determined by a method in which quantities of oxidized coenzyme Q and reduced coenzyme Q in a sample are measured by an HPLC system using a UV detector and the proportion is calculated from the quantity ratio, or by a method in which the proportion of oxidized coenzyme Q and reduced coenzyme Q is calculated from a peak area according to an HPLC system to which an electrochemical detector is attached. The system with an electrochemical detector is very useful for measuring the proportion of the reduced form existing in living bodies or samples in trace amounts, because the system can specifically measure oxidation-reduction substances and has a high sensitivity. Specifically, an electrochemical detector made by Shiseido Co., Ltd. was attached to an HPLC analytical system made by Shimadzu Corporation, and the measurement was conducted under the following HPLC conditions: column: YMC-Pack (ODS-A303), detection wavelength: 275 nm, mobile phase: methanol (88%), hexane (12%), flow rate: 1 ml/min.

Carnitine is represented as 4-trimethylamino-3-hydroxybutyric acid (4-trimethyl-3-hydroxybutyrobetaine). The carnitine derivatives such as acetyl carnitine, acyl carnitine and proprionyl carnitine can be produced by a conventionally known method such as synthesis, fermentation or extraction from a natural source. Commercially available acetyl carnitine, acyl carnitine and proprionyl carnitine products may be used as are. Yeast, dried microorganism and food which contain large amounts of carnitine derivatives may also be used.

As used herein, a derivative refers to a compound obtained by a small structural modification in a certain compound. A compound in which a hydrogen atom or a specific atomic group is substituted by another atom or another atomic group is understood to be a derivative of an original compound. In this specification, the original compound itself is also referred to as a derivative thereof.

The carnitine derivatives may be used alone or in a combination of two or more kinds. Although each carnitine derivative may be a L-form or a D-form, naturally occurring L-forms are preferred.

In the anti-fatigue composition of the present invention, the contents of reduced coenzyme Q and carnitine, acetyl carnitine, acyl carnitine or proprionyl carnitine are not particularly limited and they may be accordingly determined depending on the commercial concept of the product.

The weight ratio of reduced coenzyme Q and carnitine , acetyl carnitine, acyl carnitine or proprionyl carnitine in the present invention is not limited, but from the viewpoint of achieving a sufficient synergistic effect, the weight ratio may be 100:1 to 1:100, preferably 10:1 to 1:10, more preferably 5:1 to 1:5, most preferably 1:1.

The analysis of the weight of carnitine, acetyl carnitine, acyl carnitine or proprionyl carnitine described in this specification is conducted by a HPLC method using a fluorescent detector used for general amino acid analysis.

In the anti-fatigue composition of the present invention, the active ingredient may be a mixture of reduced coenzyme Q and oxidized coenzyme Q. The proportion of reduced coenzyme Q and oxidized coenzyme Q is not particularly limited, but from the viewpoint of achieving a sufficient synergistic effect, the proportion of the reduced coenzyme Q is preferably not less than 60% by weight, more preferably not less than 80% by weight based on total coenzyme Q. The upper limit is a maximum of 100% by weight (reduced coenzyme Q alone), and the higher the proportion of the reduced coenzyme Q, the higher the synergistic effect with carnitine, acetyl carnitine, acyl carnitine or proprionyl carnitine can be expected. However, the proportion of the reduced coenzyme Q in coenzyme Q need not be increased to the extreme, and it can be accordingly determined in consideration of the cost for stabilization and the like. The upper limit is generally not more than 99% by weight.

The reduced coenzyme Q that can be used in the present invention is those in which the number of repeating units (n in the formulas) in the side chain is 1 to 12, as represented by the above-mentioned formula (1). Of these, those containing 10 repeating units in the side chain, i.e., reduced coenzyme Q₁₀, can be suitably used from the viewpoint that sufficient effects can be obtained in human and many pets such as dogs.

The dosage form of the anti-fatigue composition of the present invention is not particularly limited, and it may be an oral preparation or a preparation applied directly to the skin. The oral preparation may be a powder preparation or may be formed into a granular preparation by adding a binder. In addition, such powder or granules maybe packed into capsules to prepare a capsule preparation.

Further, natural oil, oily higher fatty acid, higher fatty acid monoglyceride, medium-chain triglyceride (MCT), a surfactant or a mixture thereof may be added, and these oily substances may be packed as is to prepare soft capsules. In that case, capsules composed mainly of gelatin or capsules composed mainly of other water soluble polymer substances may be used. In addition, such capsules include microcapsules. Alternatively, the composition may be formed into liquid to prepare a drinkable preparation.

Pharmaceutically acceptable preparation materials other than the above-mentioned reduced coenzyme Q and carnitine derivatives may be accordingly added to the anti-fatigue composition of the present invention by a usual method. Examples thereof include, but not limited to, an excipient, a disintegrant, a lubricant, a binder, an antioxidant, a colorant, an anti-agglomeration agent, an absorption promoter, a solubilizing agent and a stabilizer.

Examples of such excipient include, but not limited to, saccharose, lactose, glucose, cornstarch, mannitol, crystalline cellulose, calcium phosphate and calcium sulfate. Examples of such disintegrant include, but not limited to, starch, agar, calcium citrate, calcium carbonate, sodium hydrogen carbonate, dextrin, crystalline cellulose, carboxymethyl cellulose and tragacanth.

Examples of such lubricant include, but not limited to, talc, magnesium stearate, polyethylene glycol, silica and hydrogenated vegetable oil. Examples of such binder include, but not limited to, ethyl cellulose, methyl cellulose, hydroxypropylmethylcellulose, tragacanth, shellac, gelatin, gum arabic, polyvinylpyrrolidone, polyvinyl alcohol, polyacrylic acid, polymethacrylic acid and sorbitol.

Examples of such antioxidant include, but not limited to, ascorbic acid, tocopherol, vitamin A, β-carotene, sodium bisulfite, sodium thiosulfate, sodium pyrosulfite and citric acid.

The above-mentioned colorant is not particularly limited and those permitted to be added to medicines may be used.

Examples of such anti-agglomeration agent include, but not limited to, stearic acid, talc, light anhydrous silicic acid and hydrous silicon dioxide.

Examples of such absorption promoter include, but not limited to, surfactants such as higher alcohols, higher fatty acids, glycerine fatty acid esters, sucrose fatty acid esters, sorbitan fatty acid esters, polyoxyethylenes, sorbitan fatty acid esters and polyglycerine fatty acid esters.

Examples of such solubilizing agent include, but not limited to, organic acids such as fumaric acid, succinic acid and malic acid. Examples of such stabilizer include, but not limited to, benzoic acid, sodium benzoate and ethyl parahydroxybenzoate.

When applying directly to the skin, the dosage form is not particularly limited, and examples of dosage form include those prepared by dissolving, or mixing and dispersing the above-mentioned medicinal substance in a suitable base, into cream, paste, jelly, gel, emulsion or liquid (ointment, liniment, lotion, cream, spray, etc.); those obtained by dissolving, or mixing and dispersing the above-mentioned medicinal substance in a suitable base and spreading the resultant on a support (poultices, etc.); and those obtained by dissolving, or mixing and dispersing the above-mentioned medicinal substance in an adhesive and spreading the resultant on a support (plaster, tape, etc.). As the base and adhesive, those usually used for medicines and cosmetics may be used according to need within the range in which the effect of the present invention is not damaged.

The anti-fatigue composition of the present invention may contain an antioxidant. Suitable examples of such antioxidant include, but not limited to, vitamin E, vitamin C, probucol, lycopene, vitamin A, carotenoids, vitamin B, flavonoids, polyphenols, glutathione, pyrroloquinoline quinone, pycnogenol, flavangenol and selenium. In addition, antioxidant enzymes may be used as an anti-oxidant. Suitable examples of antioxidant enzymes include, but not limited to, superoxide dismutase (SOD), glutathione peroxidase, glutathione-S-transferase, glutathione reductase, catalase and ascorbate peroxidase. The above-mentioned antioxidants (including antioxidant enzymes) may be used alone, or two or more of these may be mixed.

The anti-fatigue composition of the present invention may also contain other revitalizing ingredients. Suitable examples of revitalizing ingredients include, but not limited to, creatine, taurine, vitamin B₁, vitamin B derivatives and amino acids. These may be used alone, or two or more of these may be mixed. By mixing these ingredients with the coenzyme Q and carnitine , acetyl carnitine, acyl carnitine or proprionyl carnitine in the present invention, further additive or synergistic effects can be expected.

The anti-fatigue composition of the present invention may also contain a supplemental nutrient. Examples of supplemental nutrient include, but not limited to, amino acids, metal ions, saccharides, proteins, fatty acids and vitamins.

When the anti-fatigue composition of the present invention is added to food, the form include, but not limited to, edible fat and oil, cooking oil, spray oil, butter, margarine, shortening, whipping cream, concentrated milk, whiteners, dressings, pickle liquids, breads, cakes, pies, cookies, Japanese confectionaries, snacks, fried snacks, chocolates and chocolate confectionaries, rice confectionaries, roux, sauce, basting, toppings, ice creams, noodles, bread mix, fried food, processed meat products, fish paste products, frozen food such as frozen entrees, frozen meat and frozen vegetables, rice, jam, cheese, cheese food, cheese-like food, chewing gums, candies, fermented milk, canned food and drinks.

By applying the anti-fatigue composition of the present invention directly to the skin, muscle fatigue can be alleviated. In that case, the composition may also contain a substance having antiinflammatory effects. The substance having antiinflammatory effects is not particularly limited and at least one member selected from the group consisting of steroid, salicylic acid and derivatives thereof, arylacetic acid and derivatives thereof, propionic acid and derivatives thereof, fenamic acid and derivatives thereof, pyrazolone and derivatives thereof, oxicam and derivatives thereof, and non-acidic antiinflammatory agents may be used.

Examples of steroids include prednisolone valerate acetate, amcinonide, diflucortolone valerate, dexamethasone valerate, clobetasol propionate, diflorasone diacetate, dexamethasone propionate, betamethasone dipropionate, difluprednate, fluocinonide, halcinonide, budesonide, hydrocortisone butyrate propionate, betamethasone valerate, beclometasone dipropionate, fluocinolone acetonide, triamcinolone acetonide, flumetasonepivalate, hydrocortisonebutyrate, clobetasone butyrate, alclometasone dipropionate, dexamethasone, methylprednisolone acetate, prednisolone and hydrocortisone acetate.

Examples of salicylic acid derivatives include aspirin and derivatives thereof and diflunisal. Examples of arylacetic acid derivatives include indomethacin, diclofenac, sulindac, nabumetone, proglumetacin, indomethacinfarnesyl and etodolac. Examples of propionic acid derivatives include ibuprofen, naproxen, flurbiprofen, fenoprofen, tiaprofen, pranoprofen, loxoprofen and alminoprofen.

Examples of fenamic acidderivatives includemefenamic acid and tolfenamic acid. Examples of pyrazolone derivatives include phenylbutazone and oxyphenbutazone. Examples of oxicam derivatives include piroxicam, tenoxicam and ampiroxicam.

Examples of non-acidic antiinflammatory agent include epirizole, tiaramide and emorfazone. By mixing these ingredients with the anti-fatigue composition containing reduced coenzyme Q of the present invention, additive or synergistic effects can be expected.

The anti-fatigue effect encompasses effects of prevention, treatment, recovery, improvement and curing of fatigue. By administering an effective dose of the anti-fatigue composition of the present invention to a mammal, prevention, treatment, recovery, improvement and curing of fatigue of the mammal can be achieved.

By the "effective dose" is meant the amount of a compound by which an anti-fatigue effect is given to the target of treatment. The anti-fatigue effect may be objective (i.e., measurable by some test or marker) or subjective (i.e., pointed out or sensed by the target). The dosage level and the frequency of administration of specific combination of the compounds vary depending on the efficacy of each compound used, the metabolic stability and the duration of the action of the compounds, the patient's age, body weight, health condition, sex and dietary habit, the manner and time of administration, the frequency of excretion, the combination of the medicinal substances, the intensity of the symptom to be treated, and the kind of treatment that the patient receives.

The composition of the present invention may contain 0.1 to 100% by weight, preferably 1 to 99% by weight, more preferably 2 to 98% by weight of reduced coenzyme Q and carnitine, acetyl carnitine, acyl carnitine or proprionyl carnitine, and if any, oxidized coenzyme Q. The effective dose is determined within the general technical scope of those skilled in the art, who can determine the dose experimentally or empirically. The effective dose may be, for example, 0.01 mg to 1000 mg/kg body weight/day of reduced coenzyme Q and 0.01 to 1000 mg/kg body weight/day of carnitine, acetyl carnitine, acyl carnitine or proprionyl carnitine, preferably 0.01 to 500 mg/kg body weight/ day of reduced coenzyme Q and 0.01 to 500 mg/kg body weight/day of carnitine, acetyl carnitine, acyl carnitine or proprionyl carnitine, particularly 0.1 to 500 mg/kg body weight/day of reduced coenzyme Q and 0.1 to 500 mg/kg body weight/day of carnitine, acetyl carnitine, acyl carnitine or proprionyl carnitine. The effective dose may be in a single dose or in multiple doses. Although oral administration is common, non-enteral or enteral administration, for example, may be optionally selected.

The composition of the present invention may also be considered as a synergistic composition. By the term "synergistic composition" herein mentioned is meant a composition which exhibits, according to the synergistic effect of reduced coenzyme Q and carnitine, acetyl carnitine, acyl carnitine or proprionyl carnitine, activity greater than the activity expected from actions found when each is separately employed, i.e., additive activity. The synergistically effective dose of the synergistic composition may be, for example, doses of reduced coenzyme Q and carnitine, acetyl carnitine, acyl carnitine or proprionyl carnitine lower than the doses described above. However, the doses may be the same as the doses described above, which are 0.01 mg to 1000 mg/kg body weight/day of reduced coenzyme Q and 0.01 to 1000 mg/kg body weight/day of carnitine, acetyl carnitine, acyl carnitine or proprionyl carnitine, preferably 0.01 to 500 mg/kg body weight/ day of reduced coenzyme Q and 0.01 to 500 mg/kg body weight/day of carnitine, acetyl carnitine, acyl carnitine or proprionyl carnitine, particularly 0.1 to 500 mg/kg body weight/day of reduced coenzyme Q and 0.1 to 500 mg/kg body weight/day of carnitine, acetyl carnitine, acyl carnitine or proprionyl carnitine.

The present invention provides a combined preparation in which synergistically effective doses of the first active ingredient and the second active ingredient are formulated into different dosage forms, e.g., capsules, tablets or pills and the components are combined together. The present invention also provides a commercial product containing the combined preparation with a written instruction describing simultaneous or sequential application thereof where necessary, or with a package provided with an indication describing the same.

The anti-fatigue composition of the present invention can be produced by a method comprising preparing reduced coenzyme Q by an existing pharmaceutical method and mixing the reduced coenzyme Q and carnitine, acetyl carnitine, acyl carnitine or proprionyl carnitine. Alternatively, oxidized coenzyme Q and carnitine , acetyl carnitine, acyl carnitine or proprionyl carnitine may be mixed. The oxidized coenzyme Q may be converted to oxidized coenzyme Q at some point in the production process, e.g., before, at the time of, or after mixing.

When producing the anti-fatigue composition of the present invention containing reduced coenzyme Q and carnitine, acetyl carnitine, acyl carnitine or proprionyl carnitine, the content of the reduced coenzyme Q, the form of the product, the method and the form of the preservation of the product may be accordingly determined depending on the product design and the purpose of use of the anti-fatigue composition.

### Examples

Hereinafter, the present invention is described in more detail referring to Examples and Formulation Examples, but the present invention is not limited to these Examples and Formulation Examples.

### [Preparation Example 1]

### Preparation of reduced coenzyme Q₁₀

To 1000 g of ethanol were added 100 g of oxidized coenzyme Q₁₀ (purity 99.4%) and 60 g of L-ascorbic acid. The mixture was stirred at 78°C to conduct a reduction reaction. After 30 hours, the mixture was cooled to 50°C and with maintaining the temperature, 330 g of ethanol and 70 g of water were added thereto. With stirring the ethanol solution (containing 100 g of reduced coenzyme Q₁₀), the solution was cooled to 2°C at a cooling rate of 10°C/hour to obtain white slurry. The obtained slurry was filtrated under reduced pressure and the resulting wet crystal was washed with cold ethanol, cold water and cold ethanol in that order (temperature of cold solvent: 2°C), and further, the wet crystal was dried under reduced pressure (20 to 40°C, 1 to 30 mmHg) to obtain 97 g of white dry crystal. All procedures except drying under reduced pressure were carried out in a nitrogen atmosphere.

### [Preparation Example 2]

100 g of oxidized coenzyme Q₁₀ was dissolved in 1000 g of a heptane solution at 25°C. With stirring, thereto was gradually added, as a reducing agent, an aqueous solution obtained by adding 1000 g of water to 100 g of sodium hyposulfite (purity 75% or higher) and dissolving the same therein to conduct a reduction reaction at 25°C at pH 4 to 6. After 2 hours, the aqueous phase was removed from the reaction mixture and the heptane phase was washed with 1000 g of deaerated saturated saline 6 times. All procedures described above were carried out in a nitrogen atmosphere. The heptane phase was subjected to solvent replacement under reduced pressure and a 7% (w/w) ethanol solution of reduced coenzyme Q₁₀ (containing 100 g of reduced coenzyme Q₁₀) of a temperature of 50°C was prepared. To the ethanol solution was added 50 g of water and with stirring, the solution was cooled to 2°C at a cooling rate of 10°C/hour to allow crystal to be precipitated. All procedures were carried out in a nitrogen atmosphere. The obtained slurry was filtrated under reduced pressure and the resulting wet crystal was washed with cold ethanol, cold water and cold ethanol in that order (temperature of cold solvent: 2°C), and further, the wet crystal was dried under reduced pressure (20 to 40°C, 1 to 30 mmHg) to obtain 97 g of white dry crystal.

### [Example 1]

### Treadmill test using young rat (1)

Using Sprague-Dawleymale rats (7 to 10 week old, n=20), anti-fatigue effects brought about by reduced coenzyme Q₁₀ alone (about 2% of oxidized coenzyme Q₁₀ included), carnitine alone, and combination use of reduced coenzyme Q₁₀ and carnitine were evaluated with a treadmill. In the test, rats were run at a speed of 10 m/min using a treadmill apparatus (model NK-73-4 made by Natume Seisakusho Co., Ltd.), and the speed was increased stepwise, by 5 m/min every 3 minutes, and the time when the rats became unable to run (maximum running time) was measured.

As a substance to be tested, a 0.5% sodium carboxymethylcellulose (CMC-Na) aqueous solution of reduced coenzyme Q₁₀ (about 2% of oxidized coenzyme Q₁₀ included) or carnitine (reduced coenzyme Q₁₀ or carnitine/CMC-Na = 10 mg/ml) was prepared and the solution was orally administered to rats at a dose of 100 mg/kg. Rats were divided into four groups of a solvent administered group, reduced coenzyme Q₁₀ only administered group, a carnitine only administered group, and a reduced coenzyme Q₁₀ and carnitine combined group. The maximum running time was measured before and three hours after the administration and the time extended from the maximum running time was calculated. A 0.5% CMC-Na aqueous solution was administered to the control group.

**Table 1**

| | Extension of maximum running time (S) |
|---|---|
| Control group | -25±92 |
| Carnitine only administered group | -0±151 |
| Reduced coenzyme Q₁₀ only administered group | -22±71 |
| Carnitine + reduced coenzyme Q₁₀ combined group | 36±78* |

| | |
|---|---|
| * p<0.05 paired t-test | |

The maximum running time values are shown in Table 1. In the control group, the difference in the maximum running times before and after the administration of the test substance is -25±92 seconds, which suggested that the maximum running time slightly decreased. In the carnitine or reduced coenzyme Q₁₀ only administered group, no extension of the maximum running time of the rats were observed before and after the administration, but in the reduced coenzyme Q₁₀ and carnitine administered group, the maximum running times of the rats significantly increased compared to those of the control group. In other words, by combining reduced coenzyme Q₁₀ and carnitine, an anti-fatigue effect which was not apparent when they are individually used is now clearly shown, proving that the combination of these substances generates a synergistic anti-fatigue effect.

### [Example 2]

### Treadmill test using young rat (2)

Using Sprague-Dawleymale rats (7 to 10 week old, n=15), anti-fatigue effects brought about by combination use of oxidized coenzyme Q₁₀ and carnitine, and combination use of reduced coenzyme Q₁₀ and carnitine were evaluated in the same manner as in Example 1.

**Table 2**

| | Extension of maximum running time (S) |
|---|---|
| Control group | -2±22 |
| Carnitine + oxidized coenzyme Q₁₀ combined group | -12±35 |
| Carnitine + reduced coenzyme Q₁₀ combined group | 51±59** |

| | |
|---|---|
| ** p<0.01 paired t-test | |

The maximum running time values are shown in Table 2. In the case of the combination use of oxidized coenzyme Q₁₀ and carnitine, no significant extension of running time was observed, but in the case of the combination use of reduced coenzyme Q₁₀ and carnitine, significant extension of running time was observed. In other words, it has been proved that although no apparent anti-fatigue effect was found in the case of the combination use of oxidized coenzyme Q₁₀ and carnitine, an extremely strong anti-fatigue effect can be obtained in the case of the combination use of reduced coenzyme Q₁₀ and carnitine.

### [Formulation Example 1]

Olive oil was heated to 60°C and coenzyme Q₁₀ (reduced coenzyme Q₁₀: oxidized coenzyme Q₁₀= 85:15) and L-carnitine melted also at 60°C were added thereto. Vitamin E was gradually added to the mixture until homogeneous and soft capsules were prepared according to a usual method. Soft capsules containing 20 mg of coenzyme Q₁₀ and 20 mg of L-carnitine per capsule were prepared.

| | |
|---|---|
| coenzyme Q₁₀ | 20 parts by weight |
| L-carnitine | 20 parts by weight |
| vitamin E | 15 parts by weight |
| olive oil | 350 parts by weight |

### [Formulation Example 2]

Coenzyme Q₁₀ (reduced coenzyme Q₁₀: oxidized coenzyme Q₁₀= 85:15) and L-carnitine were added to acetone and the mixture was then adsorbed on crystalline cellulose (fine powder) followed by drying. The resultant was mixed with cornstarch and formed into powder according to a usual method.

| | |
|---|---|
| coenzyme Q₁₀ | 10 parts by weight |
| L-carnitine | 10 parts by weight |
| crystalline cellulose | 40 parts by weight |
| cornstarch | 55 parts by weight |

### [Formulation Example 3]

Coenzyme Q₁₀ (reduced coenzyme Q₁₀: oxidized coenzyme Q₁₀= 85:15) and L-carnitine were added to acetone and the mixture was then adsorbed on crystalline cellulose (fine powder) followed by drying. Cornstarch, lactose, carboxymethylcellulose and magnesium stearate were mixed thereto and an aqueous polyvinylpyrrolidone solution was then added thereto as a binder to form granules according to a usual method. After adding talc thereto as a lubricant and mixing, tablets containing 20 mg of coenzyme Q₁₀ and 20 mg of L-carnitine per tablet were prepared.

| | |
|---|---|
| coenzyme Q₁₀ | 20 parts by weight |
| L-carnitine | 20 parts by weight |
| cornstarch | 25 parts by weight |
| lactose | 15 parts by weight |
| carboxymethylcellulose calcium | 10 parts by weight |
| crystalline cellulose | 40 parts by weight |
| polyvinylpyrrolidone | 5 parts by weight |
| magnesium stearate | 3 parts by weight |
| talc | 10 parts by weight |

### [Formulation Example 4]

After granulating the following ingredients according to a usual method, the resultant was packed in hard gelatin capsules. Capsules containing 20 mg of coenzyme Q₁₀ and 20 mg of L-carnitine per capsule were obtained.

| | |
|---|---|
| coenzyme Q₁₀ | 20 parts by weight |
| (reduced coenzyme Q₁₀: oxidized coenzyme Q₁₀= 85:15) | |
| L-carnitine | 20 parts by weight |
| crystalline cellulose | 40 parts by weight |
| cornstarch | 20 parts by weight |
| lactose | 62 parts by weight |
| magnesium stearate | 2 parts by weight |
| polyvinylpyrrolidone | 3 parts by weight |

## Claims

1. A composition comprising:
(a) a reduced coenzyme Q represented by the following formula (1) : 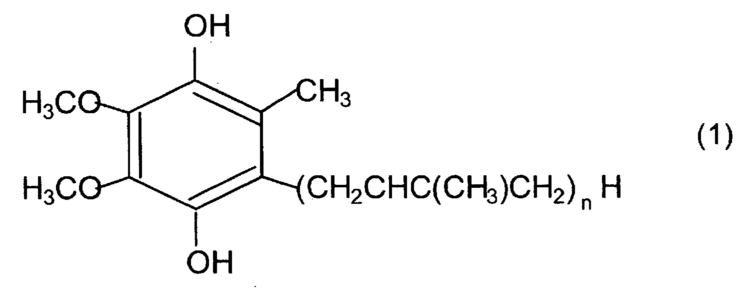 wherein n represents an integer of 1 to 12, and
(b) carnitine, acetyl carnitine, acyl carnitine or proprionyl carnitine
for use in preventing or treating fatigue in a mammal.

2. The composition according to claim 1, wherein the coenzyme Q is coenzyme Q₁₀.

3. The composition according to claim 1 as a medicine or food.

4. The composition according to claim 3, further comprising an antioxidant.

5. The composition according to claim 4, wherein the antioxidant is selected from the group consisting of vitamin C, lycopene, vitamin A, carotenoids, vitamin B, flavonoids, polyphenols, glutathione, selenium, sodium thiosulfate, vitamin E, pycnogenol, flavangenol, pyrroloquinoline quinone, superoxide dismutase (SOD), glutathione peroxidase, glutathione-S-transferase, glutathione reductase, catalase and ascorbate peroxidase.

6. Use of a composition comprising:
(a) a reduced coenzyme Q represented by the following formula (1) : 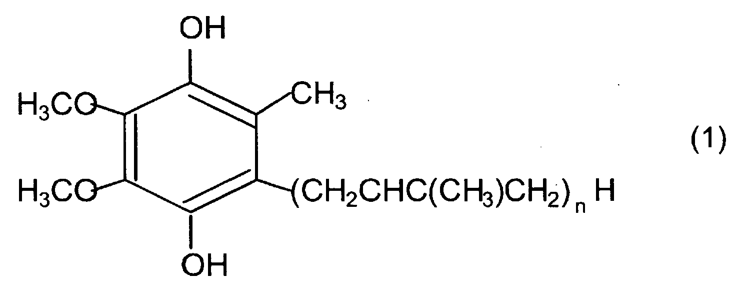 wherein n represents an integer of 1 to 12, and
(b) carnitine, acetyl carnitine, acyl carnitine or proprionyl carnitine
in the manufacture of a composition for preventing or treating fatigue in a mammal.

## Patentansprüche

1. Zusammensetzung, umfassend:
(a) ein reduziertes Coenzym Q, das durch die folgende Formel (1) dargestellt wird: 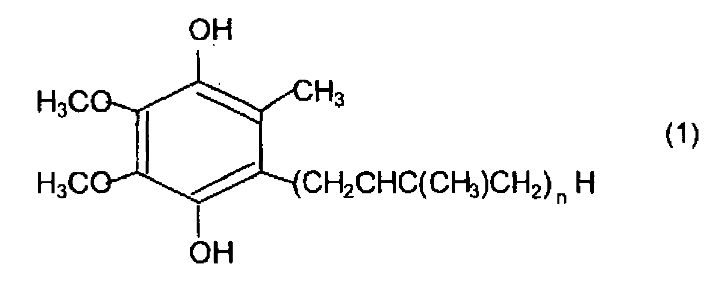 wobei n für eine ganze Zahl von 1 bis 12 steht; und
(b) Carnitin, Acetylcarnitin, Acylcarnitin oder Propionylcarnitin;
zur Verwendung zur Prävention oder Behandlung von Müdigkeit bei einem Säuger.

2. Zusammensetzung gemäß Anspruch 1, wobei es sich bei dem Coenzym Q um Coenzym Q₁₀ handelt.

3. Zusammensetzung gemäß Anspruch 1 als Medizin oder Nahrungsmittel.

4. Zusammensetzung gemäß Anspruch 3, die weiterhin ein Antioxidans umfasst.

5. Zusammensetzung gemäß Anspruch 4, wobei das Antioxidans aus der Gruppe ausgewählt ist, die aus Vitamin C, Lycopen, Vitamin A, Carotinoiden, Vitamin B, Flavonoiden, Polyphenolen, Glutathion, Selen, Natriumthiosulfat, Vitamin E, Pycnogenol, Flavangenol, Pyrrolochinolinchinon, Superoxid-Dismutase (SOD), Glutathion-Peroxidase, Glutathion-S-Transferase, Glutathion-Reductase, Catalase und Ascorbat-Peroxidase besteht.

6. Verwendung einer Zusammensetzung, umfassend:
(a) ein reduziertes Coenzym Q, das durch die folgende Formel (1) dargestellt wird: 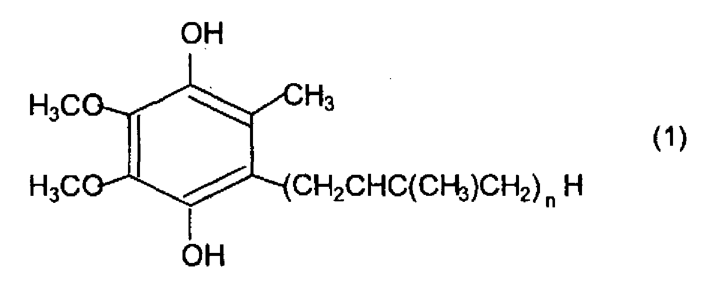 wobei n für eine ganze Zahl von 1 bis 12 steht; und
(b) Carnitin, Acetylcarnitin, Acylcarnitin oder Propionylcarnitin;
bei der Herstellung einer Zusammensetzung zur Prävention oder Behandlung von Müdigkeit bei einem Säuger.

## Revendications

1. Composition comprenant :
(a) une coenzyme Q réduite représentée par la formule (1) suivante: 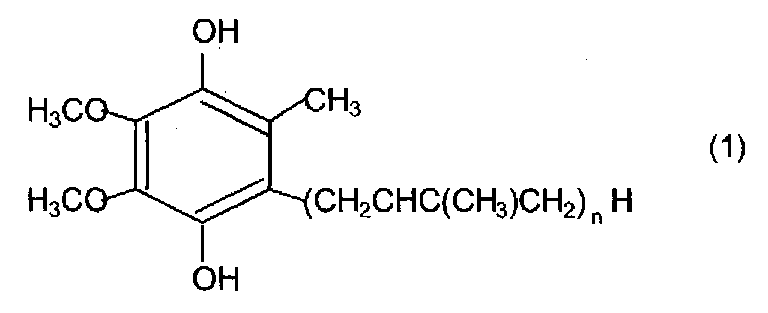 où n représente un entier de 1 à 12, et
(b) de la carnitine, de l'acétylcarnitine, de l'acylcarnitine ou de la proprionylcarnitine
pour utilisation dans la prévention ou le traitement de la fatigue chez un mammifère.

2. Composition selon la revendication 1, où la coenzyme Q est la coenzyme Q₁₀.

3. Composition selon la revendication 1 en tant que médicament ou aliment.

4. Composition selon la revendication 3, comprenant en outre un antioxydant.

5. Composition selon la revendication 4, où l'antioxydant est sélectionné dans le groupe constitué de la vitamine C, de lycopène, de la vitamine A, de caroténoïdes, de la vitamine B, de flavonoïdes, de polyphénols, de glutathione, de sélénium, de thiosulfate de sodium, de vitamine E, de pycnogénol, de flavangénol, de pyrroloquinoléinequinone, de superoxyde dismutase (SOD), de glutathione peroxydase, de glutathione-S-transférase, de glutathione réductase, de catalase et d'ascorbate peroxydase.

6. Utilisation d'une composition comprenant :
(a) une coenzyme Q réduite représentée par la formule (1) suivante : 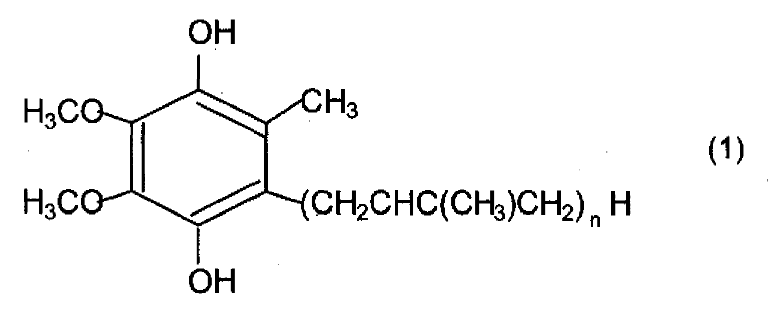 où n représente un entier de 1 à 12, et
(b) de la carnitine, de l'acétylcarnitine, de l'acylcarnitine ou de la proprionylcarnitine
pour la préparation d'une composition destinée à prévenir ou traiter la fatigue chez un mammifère.
